# NEW EUROPEAN PATENT SPECIFICATION

(11) **EP 1 296 672 B2**
(45) Date of publication and mention of the opposition decision: **24.10.2018**
(45) Mention of the grant of the patent: 31.12.2008
(21) Application number: 00935420.0
(22) Date of filing: 09.06.2000
(51) Int. Cl.: A61K 31/22, A61K 31/366, A61K 31/404, A61K 47/02

(54) **STABLE PHARMACEUTICAL PRODUCT AND FORMULATION**
STABILISIERTES ARZNEIMITTEL UND ARZNEIZUBEREITUNG
PRODUIT ET PREPARATION PHARMACEUTIQUES STABLES

(43) Date of publication of application: 02.04.2003
(73) Proprietor: LEK Pharmaceuticals d.d., 1526 Ljubljana (SI)
(72) Inventor: PFLAUM, Zlatko, 1230 Domzale (SI); MILIVOJEVIC, Dusan, 1000 Ljubljana (SI); RUCMAN, Boris, 1211 Ljubljana (SI); KOGEJ, Stojan, 1260 Ljubljana (SI)
(74) Representative: TBK
(86) International application number: PCT/IB2000/000771
(87) International publication number: WO 2001/093859

(56) References cited:
- EP-A- 0 336 298
- EP-A- 0 547 000
- EP-A1- 0 837 069
- WO-A-00/21525
- WO-A1-00/12088
- WO-A1-00/35425
- WO-A1-94/16693
- WO-A1-95/25045
- WO-A1-95/34488
- WO-A1-96/04189
- WO-A2-99/06035
- US-A- 4 753 352
- US-A- 5 114 003
- US-A- 5 225 202
- Physician's Desk Reference, "PDR", 1995, Ed. 49
- Package insert of Pravachol® (Pravastatin Sodium tablets), Bristol-Myers Squibb, Princeton, NJ. Apr. 1997
- KLAMM S.W. ET L: 'Sample volume measurement errors caused by CO² adsorption in desiccants' ENVIRON. SCI. TECHNOL. vol. 23, 1989, pages 1420 - 1422
- CARTER S.J.: 'Tutorial Pharmacy', vol. 6TH ED., 1972, PITMAN BOOKS LIMITED, LONDON pages 48 - 49
- United States Pharmacopeial Convention Inc., "The United States Pharmacopeia (USP XXII, NFXVII), The National Formulary", 1990, 22nd Ed.
- MERSMANN A.: 'Adsorption', vol. B3, 1988, ULLMANN'S ENCYCLOPEDIA OF INDUSTRIAL CHEM. pages 9-1 - 9-52
- Material safety data sheet for lithium hydroxide (Sigma Chemical Co.)
- DUCROS: 'Utilisation de l'hydroxyde de lithium comme absorbant de l'anhydride carbonique' REVUE DES CORPS DE SANTÉ vol. 7, no. 4, 1966, page 603
- SKOOG AND WEST: 'Fundamentals of Analytical Chemistry', vol. 2ND ED., 1973, CLARKE, DOBLE & BRENDON LTD. page 301
- Material safety data sheet for potassium supèroxide (Sigma Chemical Co.)
- Material safety data sheet for potassium supéroxide (https://fscimage.fishersci.com/msds/00075. html)
- Material safety data sheet for potassium supéroxide (Acros Organics)
- Extract from the Fisher scientific catalogue, 1998
- DICKSON A.G. ET AL: 'Handbook of Methods for the Analysis of the Various Parameters of the Carbon Dioxide System in Sea Water', vol. VERS. 2, September 1994

## Description

### Field of the invention

The present invention relates to a new concept of stabilizing a HMG-CoA reductase inhibitor which is used in a pharmaceutical formulation being particularly suitable for the treatment of hypercholesterolemia and hyperlipidemia. More precisely, the present invention relates to the use of a substance capable of binding and/or neutralizing carbon dioxide in a pharmaceutical packaging as defined in the claims.

### Background of the Invention

Loyastatin, pravastatin, simvastatin, mevastatin, atorvastatin, fluvastatin and cerivastatin, derivatives and analogs thereof are known as HMG-CoA reductase inhibitors and are used as antihypercholesterolemic agents. The majority of them are produced by fermentation using microorganisms of different species identified as species belonging to *Aspergillus, Monascus, Nocardia, Amycolatopsis, Mucor* or *Penicillium* genus. Some are obtained by treating the fermentation products using the methods of chemical synthesis like simvastatin or they are the products of total chemical synthesis like fluvastatin, atorvastatin and cerivastatin.

The purity of the HMG-CoA reductase inhibitor as the active substance is an important factor for manufacturing a safe and effective pharmaceutical formulation. Maximum possible purity of the product is of particular importance if the pharmaceutical product must be taken on a longer term basis in the treatment or prevention of high cholesterol levels in blood. Accumulation of impurities from drugs of a lower level of purity may cause a variety of side effects during treatment. Besides impurities, that cannot be completely eliminated in the process of preparation of the active substance, degradation products occurring by subjecting the final pharmaceutical formulation to various environmental factors such as temperature, moisture, low pH and light, may also impose a problem. HMG-CoA reductase inhibitors occurring in the form of salts in the final pharmaceutical formulation, such as atorvastatin, pravastatin, fluvastatin and cerivastatin, are particularly sensitive to an acidic environment in which hydroxy acids are degraded into a lactone.

Apart from the fact that the aforementioned active substance may be destabilised by the environmental factors, their degradation may also be accelerated by interactions with other pharmaceutical ingredients, such as fillers, binders, lubricants, glidants and disintegrating agents. Therefore, the pharmaceutical ingredients and the process for preparation of the pharmaceutical formulation should be meticulously chosen to avoid the aforementioned undesired interactions and reactions.

The stability of the active substance in an acidic environment is one of the major problems in the case of statins in the form of salts. For example, the sodium salt of pravastatin provides a pH between 7 and 8.5, normally around 7.5, when brought into a water solution at a concentration of 5 wt.-%. At this pH, not only the solid form but also dissolved pravastatin Na slowly converts into pravastatin lactone, which is to be considered as impurity and degradation product. As a possible solution of this problem, EP 0 336 298 proposes a stable pharmaceutical formulation for pravastatin. The essence of the formulation is to maintain an alkaline environment so that the aqueous dispersion of the pharmaceutical formulation reaches a pH above 9, preferably about 10. In addition to the active substance pravastatin, the composition of the invention includes a basifying agent, such as magnesium oxide which imparts a pH to an aqueous dispersion of the aforementioned formulation above 9. In view of the stability of the active substance such a formulation is effective. However, the local alkaline environment occurring at the site of dissolution of the pharmaceutical formulation may have a negative impact on the gastric mucosa with its normally acidic environment. This negative impact may be particularly evident for patients with a damaged gastric mucous membrane where the mucosa per se is not able to create a sufficient acidic environment inside the stomach for normal digestive functioning. It is particularly important in chronic therapies as in the case of prophylaxis or treatment with HMG-CoA reductase inhibitors.

Other approaches for providing a stable pharmaceutical formulation are described in the present Applicant's earlier PCT application No. PCT/IB99/01749, as well as in the present Applicant's PCT application of even date.

EP-A-0 547 000 discloses fluvastatin, sodium comprising compositions containing a basifying agent.

WO 00 21525 A discloses a composition comprising fluvastatin sodium and potassum bicarbonate.

US-A-5 225 202 discloses enteric coated pellets containing pravastatin sodium with sodium citrate in its core and sodium hydroxide in the over coat.

### Summary of the Invention

According to the present invention, there is provided a use of a substance capable of binding and/or neutralizing carbon dioxide as defined in claim 1.

In the inventor's investigations, it was found that carbon dioxide from the air can bind, normally partly reversible and partly irreversible, to the HMG-CoA reductase inhibitor as the active substance. This binding of carbon dioxide can cause a drop of pH within the HMG-CoA reductase inhibitor substance in bulk or within the HMG-CoA reductase inhibitor-containing pharmaceutical formulation and, thus, represents a major reason for instability problems in case of a pharmaceutical formulation containing an active substance and in case of a bulk active substance. Even if a non-stabilized HMG-CoA reductase inhibitor-containing pharmaceutical formulation is packed under nitrogen atmosphere but was before exposed to normal atmosphere, the pH which would be generated when obtaining an aqueous solution thereof is slowly going down and eventually leads to an increase in impurities and degradation products. By means of the particular combination of a HMG-CoA reductase inhibitor and a substance capable of binding and/or neutralizing carbon dioxide in accordance with the present invention, a strong protective effect is achieved.

With addition of carbon dioxide binding and/or neutralizing substance, the pH decrease is effectively lowered or ceased. The carbon dioxide binding and/or neutralizing substance can remove the carbon oxide which may already be present in the HMG-CoA reductase inhibitor as such (in bulk) before being formulated into the pharmaceutical formulation, but can also remove the carbon dioxide which may be present in, and which may pass into the packaging such as bags or bottles.

Furthermore, by using the carbon dioxide binding and/or neutralizing substance according to the present invention, the final pharmaceutical packaging is highly resistant to the negative effects of both carbon dioxide from the air, and a much better protection against low pH conditions is achieved.

### Brief description of the drawings

Figures 1a and 1b show HPLC chromatograms of unstabilized pravastatin Na (Fig. 1a) and of unstabilized pravastatin Na after the exposure to carbon dioxide atmosphere (Fig. 1b), showing the formation of different degradation products (impurities) of unstabilized and unprotected pravastatin.
Figure 2 is a graph which shows the change or the maintenance of pH over time when a sample of unstabilized pravastatin is either protected against carbon dioxide according to the invention or not protected when exposed to air.
Figure 3 is a graph which shows the change or the maintenance of pH over time when a sample of stabilized pravastatin is either protected against carbon dioxide according to the invention or not protected when exposed to carbon dioxide atmosphere.

### Detailed description of the preferred embodiments

In the present invention, we have surprisingly found that a sufficient stability of the active substance, which is a HMG-CoA reductase inhibitor preferably in the form of salt, can be also obtained by using an approach which does not require basifying the active substance-containing pharmaceutical formulation to provide a pH of at least 9 and thus does not create a marked alkaline environment in an aqueous solution or dispersion of the pharmaceutical formulation. In order to achieve this efficiently, it is significant that the HMG-CoA reductase inhibitor is combined with the carbon dioxide binding and/or neutralizing substance.

The carbon dioxide binding and/or neutralizing substance is provided in an element of a packaging which element is separated from the element or compartment containing the HMG-CoA reductase inhibitor. This is suitable for carbon dioxide binding and/or neutralizing substances which are not pharmaceutically acceptable. This is advantageous, because pharmaceuticall inacceptable substances are known which bind, neutralize and/or react with carbon dioxide in a very strong and fast manner, such as molecular sieves, superoxides or alkali metal hydroxides. Carbon dioxide can thus be removed from the atmosphere which is present in the packaging in a very efficient way. In a further embodiment, aforementioned separate combination with the carbon dioxide binding and/or neutralizing substance may also be provided in an intermediate product during the manufacture of, or after the isolation and purification of the HMG-CoA reductase inhibitor. With this concept, it is possible to achieve sufficient protection from the negative impact of carbon dioxide during the manufacturing, during the handling and during the shipping of the HMG-CoA reductase inhibitor compound as such. Accordingly, in a further embodiment the HMG-CoA reductase inhibitor and the carbon dioxide binding and/or neutralizing substance may be respectively contained in separate elements of a storing or shipping package.

If not already protected or if further protection against carbon dioxide is desired, according to the present invention both the HMG-CoA reductase inhibitor, which is incorporated into a pharmaceutical formulation together with appropriate further additives to provide at least one pharmaceutical formulation unit, and the aforementioned carbon dioxide binding and/or neutralizing substance are provided in separate elements or compartments of a pharmaceutical packaging. The kind and shape of the pharmaceutical formulation unit and the elements or compartments containing it, of the elements or compartments containing the carbon dioxide binding and/or neutralizing substance, and of the pharmaceutical packaging is not critical and may be designed as desired. The pharmaceutical formulation unit usually is a solid pharmaceutical dosage form such as compressed tablet or capsules. The solid pharmaceutical dosage form such as tablets or capsules may be placed into appropriate dosage form containers to form the pharmaceutical packaging, such as plastic bags, blister packages metal bags or glass bottles. The carbon dioxide binding and/or neutralizing substance may be placed in an appropriate separate element or compartment within the pharmaceutical packaging, such as a porous or perforated body, a gas permeable bag or jar, a permeable or semipermeable membrane, or the like.

The pharmaceutical packaging which contains one or more formulations of a HMG-CoA reductase inhibitor can be suitably produced by a process comprising the step of incorporating the substance capable of binding and/or neutralizing carbon dioxide into the pharmaceutical packaging.

Furthermore, in stablising the pharmaceutical packaging according to the present invention, the separate element or compartment containing the carbon dioxide binding and/or neutralizing substance may be incorporated into the pharmaceutical packaging under nitrogen atmosphere in order to further reduce carbon oxide content.

In the pharmaceutical packaging while the element containing the carbon dioxide binding and/or neutralizing substance is permeable or semipermeable to the environment inside the product or the pharmaceutical packaging is preferably sealed or airtightly closed to prevent further negative impact from the carbon dioxide of the outside air.

The HMG-CoA reductase inhibitor will now be described in further detail. This pharmaceutically active substance is selected from the group consisting of pravastatin, atorvastatin, fluvastatin, cerivastatin and a pharmaceutically acceptable salt thereof. Preferably, the active substance is in the form of a solid salt. The resistance against carbon dioxide is particularly effective when the HMG-CoA reductase inhibitor is the sodium salt of pravastatin (pravastatin Na) or the calcium salt of atorvastatin (atorvastatin Ca). Nontheless, the stabilizing effect is also achieved when using other HMG-CoA reductase inhibitors.

In preferred embodiments, the HMG-CoA reductase inhibitor and/or the pharmaceutical formulation is/are further stabilized against moisture and pH sensitivity. We have found that for the stability and digestibility of a pharmaceutical formulation both the pH generated by the formulation in an aqueous medium (usually being a dispersion) and the pH of the active substance (HMG-CoA reductase inhibitor) are preferably adjusted as described by the present Applicants' earlier application PCT/IB/01749. As described therein, a high stability of the HMG-CoA reductase inhibitor is obtained with an active substance which is capable of providing a pH in the range from 7 to 12 and preferably from 8 to 11. On the other hand, the pharmaceutical formulation does not require a marked alkaline condition, and in order to avoid problems of digestability as in the prior art, the pH of the formulation is preferably adjusted to below 9, preferably to a range between 6 and 9 and particularly between 7 and 8.5. The pH value is the one which is obtained when the pH of an aqueous medium containing said active substance or the formulation would be measured.

The preferred additional stabilization is suitably effected by the addition of buffering or basifying substances to the HMG-CoA reductase inhibitor in bulk, or by the addition of buffering or basifying substances to the pharmaceutical formulation, as described in aforementioned application PCT/IB/01749 and in the present Applicants' PCT application.

When added to the HMG-CoA reductase inhibitor in bulk, the amount of buffering agent or basifying substance may be relatively small. Accordingly, the active substance may contain a buffering agent or a basifying substance at an amount ratio of the buffering substance or the basifying substance of 30 wt.-% or below, preferably 10 wt.-% or below, more preferably 5 wt.-% or below and in particular 1 wt.-% or below, relative to the amount of HMG-CoA reductase inhibitor. The preferred lower limit mainly depends on the environmental conditions and the kind and amounts of other components to be used for the pharmaceutical formulation, but an amount of at least 0.05 or 0.1 wt.-% of the buffering substance or the basifying substance, relative to the amount of HMG-CoA reductase inhibitor, may be used. For example, an amount 0.3% of sodium carbonate in pravastatin results in a pH of pravastatin between 9 and 10. Thus, it is possible to mix HMG-CoA reductase inhibitor like pravastatin with other ingredients of the pharmaceutical formulation without fear that a degradation can be caused by the contact of pravastatin with acidic ingredients as a microenvironment of pravastatin is still basic due to the addition of small amounts of a buffering agent.

When added to the pharmaceutical formulation as desired, optionally in addition to the admixture or the homogeneous composition with the HMG-CoA reductase inhibitor in bulk, the buffering agent or basifying substance may be used in amounts of 20% or less, preferably 10% per weight or less based on the total weight of the pharmaceutical formulation.

If used, any buffering or basifying substance capable of adjusting the pH of the total formulation in the desired range is suitable. The buffering substance or agent is suitably selected from the group consisting of salts of inorganic acids, salts of organic bases or salts of organic acids. Examples of salts of inorganic acids include sodium or potassium citrate; sodium or potassium phosphate or hydrogen phosphate, dibasic sodium phosphate, sodium, potassium, magnesium or calcium carbonate or hydrogen carbonate, sulphate, or mixtures of such buffering agents, or the like; carbonate buffer or phosphate buffer, such as sodium carbonate of sodium phosphate, being preferred. Examples for salts of organic bases include aminoguanidine carbonate or hydrogen carbonate, guanidine carbonate or hydrogen carbonate, succinimide carbonate or hydrogen carbonate, 1-adamantil amine carbonate or hydrogen carbonate, N,N'-bis(2-hydroxyethyl) ethylendiamine carbonate or hydrogen carbonate, tris (hydroxymethyl) aminometan carbonate or hydrogen carbonate, D(-)-N-Methylglucamine carbonate or hydrogen carbonate, or the like. Examples for salts of organic acids include potassium or sodium salts of acetic acid, citric acid, lactic acid, ascorbic acid, maleic acid, phenylacetic acid, benzoic acid, lauryl sulphuric acid, or the like. The basifying substance or agent is suitable selected from the group consisting of metal oxides, inorganic bases, organic bases and organic acids with basic character. Examples of metal oxides include magnesium oxide and aluminum oxide. Examples of inorganic bases include alkali metal hydroxide such as sodium hydroxide, potassium hydroxide, alkali earth metal hydroxide such as calcium hydroxide or magnesium hydroxide. Examples of organic bases include succinimide, 1-adamantyl amine, N,N'-bis(2-hydroxyethyl) ethylendiamine, tris (hydroxymethyl) aminomethan, D(-)-N-methylglucamine, or the like. Examples of organic acids with basic character include 3-(N-morpholino)propanesulfonic acid, 4-[cyclohexyl amino]-1-butansulfonic acid, 4-[cyclohexyl amino]-1-etansulfonic acid, the salts of these organic acids with alkaline or alkaline earth metals, and further include arginine, ornithine, lysine, or the like.

The carbon dioxide binding and/or neutralizing substance utilized in the present invention will now be described in further detail. The concept of binding and/or neutralizing includes any possibility of removing carbon dioxide or preventing it from interacting with the HMG-CoA reductase inhibitor. Accordingly, this substance may be selected from the group of compounds which are effective for adsorbing, absorbing and/or neutralizing carbon dioxide and/or reacting therewith, or any combination of these measures. The affinity of the substance used towards carbon dioxide by means of binding, neutralizing and/or reacting is preferably much higher than the respective affinity of HMG-CoA reductase inhibitor like pravastatin Na towards carbon dioxide in order to provide a significant protective effect.

Compounds for adsorbing includes hydrophobic adsorbers, i.e., activated carbon and preferably hydrophilic adsorbers, i.e., zeolites and activated aluminum oxide and Fuller's earth. The adsorbing compounds have appropriate pores for effectively adsorbing carbon dioxide, as described, for example, in "Ullmann's Encyclopedia of Industrial Chemistry", 5th edition (1988), Vol. B-3, Chapter 9. Preferred adsorbing compounds are zeolites, and particularly the zeolite types 5A, 4A, 10X and 13X.

Compounds for absorbing include alkali metal hydroxides or alkali metal superoxides. Particularly preferred are potassium superoxide and the alkali metal hydroxides KOH, NaOH and LiOH.

Compounds for neutralizing, and possibly also absorbing and/or reacting with, carbon dioxide includes appropriate buffering substances and appropriate basifying substances, such as the buffering and basifying substances mentioned above as being also effective for adjusting the pH as defined in claim 1. For neutralizing carbon dioxide, alkali metal hydroxides, alkali metal carbonates and alkali metal bicarbonates are particularly effective. Since buffering substances per se do not have, in comparison with other carbon dioxide binding substances mentioned above, such a strong affinity towards binding and/or neutralizing carbon dioxide from the atmosphere within the packaging, it is preferred that such buffering substances are physically mixed directly with the HMG-CoA reductase inhibitor, especially with this active substance itself (in bulk) as already described above. In this case, a stabilization against the negative influences of pH and moisture and a protection against carbon dioxide are achieved at the same time. An efficient overall protection against various negative influencing factors can be obtained when a buffering or basifying substance is mixed in a physical manner with the HMG-CoA reductase inhibitor in bulk or with the pharmaceutical formulation, and in addition another substance which removes carbon dioxide from the atmosphere present in the pharmaceutical packaging is used in a separate element thereof as described above.

Thus, the above mentioned carbon dioxide binding and/or neutralizing substances may be used alone or in combination. Furthermore, the binding of moisture or the reaction or co-reaction with water vapor may occur at the same time. The forms of the substances are not critical and may dependent on the kind of material used and the design of the packaging as desired. For example, the substances may be in the form of powders, granules, spheres. pellets, tablets, rods, or other moldings or pressed forms. The substances may be incorporated into the packaging for example by means of bags or similar elements, but they may be also placed into perforated or permeable bodies which are connected to parts or elements of the packaging such as the screw cap of bottles. If possible in view of the kind of substance, the substance itself may also form at least a part of the packaging material, e.g. when using carbon dioxide binding polymer membranes.

By following the concepts of the present invention, the HMG-CoA reductase inhibitor as well as the pharmaceutical formulation containing it as the active substance are stable by being resistant against the negative effect of carbon dioxide and do not tend to be decomposed and, thus, essentially retain their activity.

The pharmaceutical formulation used in the invention may include, in addition to the HMG-CoA reductase inhibitor, one or more fillers, such as microcrystalline cellulose, lactose, sugars, starches, modified starch, mannitol, sorbitol and other polyols, dextrin, dextran and maltodextrin, calcium carbonate, calcium phosphate and/or hydrogen phosphate, sulphate, one or more binders, such as lactose, starches, modified starch, dextrin, dextran and maltodextrin, microcrystalline cellulose, sugars, polyethylene glycols, hydroxypropyl cellulose, hydroxypropyl methylcellulose, ethylcellulose, hydroxyethyl cellulose, methylcellulose, carboxymethyl cellulose, gelatin, acacia gum, tragacanth, polyvinylpyrrolidone, magnesium aluminium silicate, one or more disintegrating agents such as croscarmellose sodium, cross-linked polyvinylpyrrolidone, cross-linked carboxymethyl starch, starches and microcrystalline cellulose, magnesium aluminium silicate, polyacrylin potassium, one or more different glidants such as magnesium stearate, calcium stearate, zinc stearate, calcium behenate, sodium stearyl fumarate, talc, magnesium trisilicate, stearic acid, palmitic acid, carnauba wax, silicon dioxide, one or more buffering agents such as sodium or potassium citrate, sodium phosphate, dibasic sodium phosphate, calcium carbonate, hydrogen phosphate, phosphate, sulphate, sodium or magnesium carbonate, sodium ascorbinate, benzoate, sodium or potassium hydrogen carbonate, lauryl sulphate, or any mixtures of these.

If required any, the formulation may also include surfactants and other conventional components for solid, pharmaceutical formulations such as colouring agents, lakes, aromas and adsorbents. As surfactants the following may be used: ionic surfactants, such as sodium lauryl sulphate or non-ionic surfactants such as different poloxamers (polyoxyethylene and polyoxypropylene copolymers), natural or synthesized lecithins, esters of sorbitan and fatty acids (such as Span®, manufactured by Atlas Chemie), esters of polyoxyethylenesorbitan and fatty acids (such as Tween®, manufactured by Atlas Chemie), polyoxyethylated hydrogenated castor oil (such as Cremophor®, manufactured by BASF), polyoxyethylene stearates (such as Brij®, manufactured by Atlas Chemie), dimethylpolysiloxane or any combination of the above mentioned surfactants.

If the solid pharmaceutical formulation is in the form of coated tablets, the coating may be prepared from at least one film-former such as hydroxypropyl methylcellulose, hydroxypropyl cellulose, at least from one plasticizer such as polyethylene glycols, dibutyl sebacate, triethyl citrate, and other pharmaceutical auxiliary substances conventional for film coatings, such as pigments, fillers and others.

The solid pharmaceutical formulations may be prepared as described below:
- The mixture of the active substance, filler, binder, buffering agent, disintegrating agent and if required a surfactant and other conventional ingredients for solid pharmaceutical formulations is homogenised employing suitable mixers. Glidants and/or lubricants are added and the mixture is re-homogenised. The resulting mixture is compressed into tablets or filled into capsules. If needed, tablets can be film-coated.
- The mixture of the active substance, filler, binder, buffering agent, disintegrating agent and if required a surfactant and other conventional ingredients for solid pharmaceutical formulations is homogenised employing suitable mixers, granulated with a suitable solvent such as water, ethanol, methanol, isopropyl alcohol, n-butyl alcohol, acetone, diethyl ether, ethyl acetate, isopropyl acetate, methyl acetate, dichloromethane and methanol, and mixtures of these solvents such as ethanol and acetone, methanol and acetone, dichloromethane and methanol, and the mixtures thereof. The resulting granulation is dried in suitable dryers such as standard plate dryers, fluid bed dryers, vacuum and microwave dryers. To the dried granulation, glidants and/or lubricants and if required other conventional ingredients for solid pharmaceutical formulations are added. The resulting mixture is rehomogenised and compressed into tablets or filled into capsules. Optionally, tablets are film-coated.

The present invention is illustrated but by no means limited by the following examples.

### EXAMPLES

The following experiments were done to show the influence of carbon dioxide from the air on the formation of degradation products (impurities) and on the change of pH of pravastatin Na as a representative HMG-CoA reductase inhibitor and to show the improved stability of pravastatin Na in the presence of substances that can bind and/or neutralize carbon dioxide. Pravastatin Na stabilised by the admixture or composition with buffering or basifying substances as described in the Applicants' earlier application PCT/IB99/01749 and the Applicants' co-pending application of even date is very stable and does not show significant change of pH in case of exposure to carbon dioxide from the air. Therefore the samples, in case of experiments where the correspondingly stabilised pravastatin was used, were exposed to the atmosphere of pure carbon dioxide, so the changes in pH become evident in a relatively short period of time such as a day or two.

### 1. Degradation of HMG-CoA reductase inhibitor

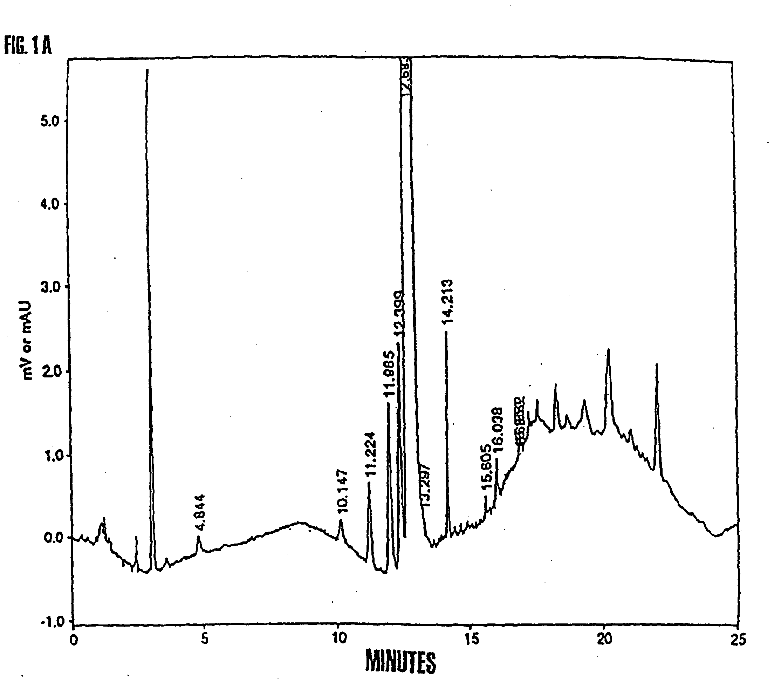

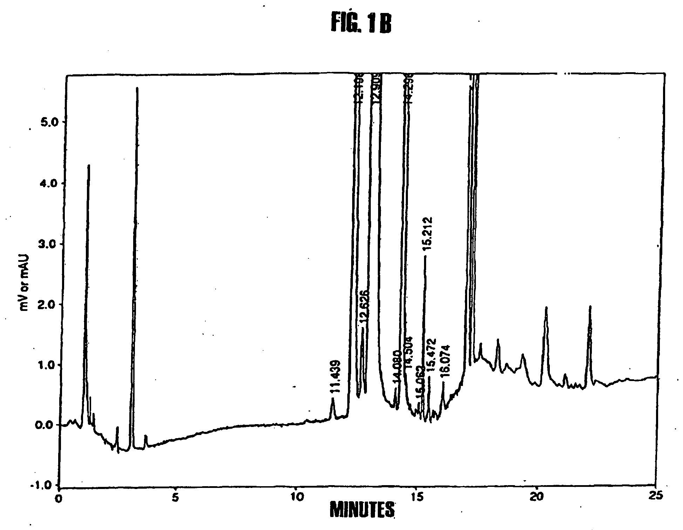

In Fig. 1a, a HPLC chromatogram of a freshly perpared sample of pravastating Na is shown. The same pravastating Na was exposed to carbon dioxide atmosphere for one hour, and then a further HPLC chromatogram was prepared (Fig. 1b). From these HPLC chromatograms it is evident that in case of exposure of pravastatin Na to carbon dioxide two different peaks in front of and three peaks after the peak of pravastatin Na appear. These peaks correspond to degradation products of the HMG-CoA reductase inhibitor.

### 2. Carbon dioxide protection with unstabilized HMG-CoA reductase inhibitor

Crystals of pravastatin Na were prepared as follows: The sodium salt of pravastatin (1 g) was dissolved in methanol (10 ml) and while stirring ethyl acetate was added. The resulting clear yellow solution was cooled to 8°C and allowed to stand overnight. Formed radiating clusters of thin, long needle-like crystals were filtered, washed with ethyl acetate (20 ml) and dried. Yield: 0.87 g of pale yellow crystals, melting point 172 - 174°C. Crystals have pH 8.3.

Pravastatin Na crystals were exposed to air atmosphere in:
1a) opened glass bottle protected only against light
1b) closed polyethylene bag
1c) closed polyethylene bag with addition of KO₂ in separate jar

### 3. Carbon dioxide protection with stabilized HMG-CoA reductase inhibitor

After one day when the pH drop to 7.6 the pravastatin Na crystals were re-crystallised with addition of buffers. First 5 g of pravastatin Na was dissolved in 30 ml of MeOH and then
A 0,2% (w/w) Na₂CO₃,
B 0,25% (w/w) Na₂HPO₄
was added. The crystals were formed by addition of 400 ml of ethyl acetate containing 2% of water.

Pravastatin Na crystals stabilized by the addition of buffering substance were exposed to pure carbon dioxide in:
2a) opened glass bottle protected only against light in carbon dioxide atmosphere
2b) closed polyethylene bag
2c) closed polyethylene bag with addition of KOH in separate jar
2d) closed polyethylene bag with addition of KO₂ in separate jar

### 4. Results and Observations with Carbon Dioxide Protection

The pH was measured in case of stabilised pravastatin Na in 1% aqueous solution (lower amount of material necessary) and in case of unstabilized pravastatin Na in 5% aqueous solution.

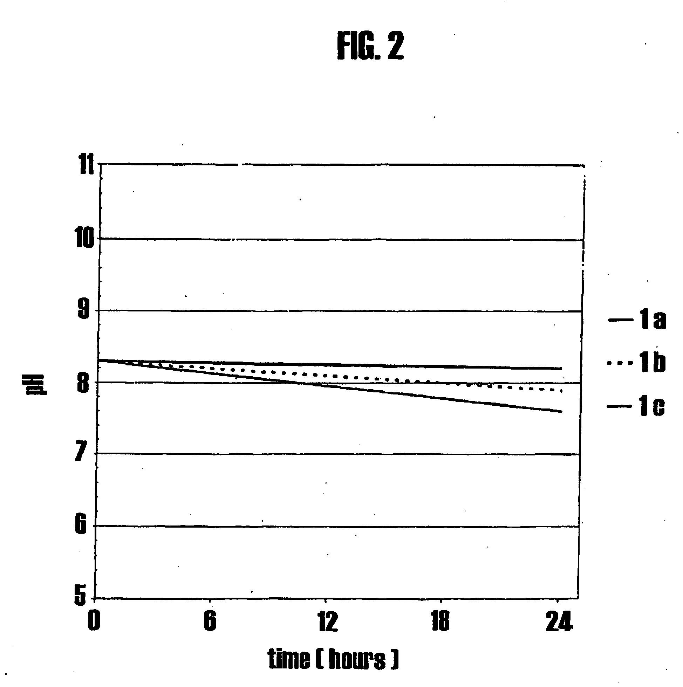

The results for unstabilized pravastatin Na exposed to air is shown in the following Table 1 and illustrated graphically in Fig. 2.

**Table 1**

| time (hours) | 1a | 1b | 1c |
|---|---|---|---|
| 0 | 8.3 | 8.3 | 8.3 |
| 24 | 7.6 | 7.9 | 8.2 |

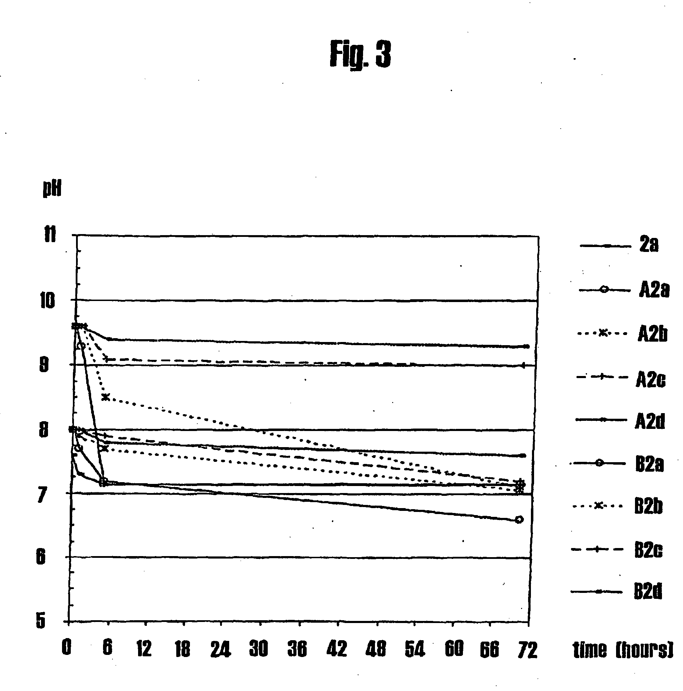

The results for stabilised pravastatin Na exposed to carbon dioxide atmosphere is shown in the following Table 2 and illustrated graphically in Fig. 3.

**Table 2**

| | | pH | | | | pH | | | |
|---|---|---|---|---|---|---|---|---|---|
| | | A | | | | B | | | |
| time (h) | 2a * | A2a | A2b | A2c | A2d | B2a | B2b | B2c | B2d |
| 0 | 7.60 | 9.60 | 9.60 | 9.60 | 9.60 | 8.00 | 8.00 | 8.00 | 8.00 |
| 1 | 7.30 | 9.30 | 9.60 | 9.60 | 9.60 | 7.70 | 7.90 | 8.00 | 8.00 |
| 5 | 7.14 | 7.15 | 8.50 | 9.10 | 9.40 | 7.20 | 7.70 | 7.90 | 7.80 |
| 70 | 7.14 | 7.15 | 7.10 | 9.00 | 9.30 | 6.60 | 7.05 | 7.20 | 7.60 |
| * unstabilized pravastatin Na | | | | | | | | | |

It is evident that in case of unstabilized pravastatin Na, even if it is packed under nitrogen atmosphere but was before that exposed to normal atmosphere, pH is slowly going down. With addition of carbon dioxide binding and/or neutralizing substance the pH decreases only slightly, see sample 1c. This is the result of binding of the carbon dioxide brought with pravastatin Na into the packing, and that carbon dioxide can not be removed only with the replacement of air atmosphere with nitrogen atmosphere. The carbon dioxide binding and/or neutralizing substances removes also the carbon dioxide which can pass in to the packaging material such as polyethylene bags, metal bags, screw cap bottles.

Pravastatin sodium, which is protected from carbon dioxide by the direct incorporation of a buffering substance into the HMG-CoA reductase inhibitor in bulk, is very stabile in normal atmosphere (no change of pH in one week, not shown). In case of its exposure to carbon dioxide atmosphere the pH change of samples with KOH and KO₂ was significantly reduced in comparison with the non-protected samples, as shown by the Tables above and Figs. 2 and 3.

Instead of the HMG-CoA reductase inhibitor itself as used in the above experiments, pharmaceutical formulations containing the same can be used for being protected against carbon dioxide. Using typical amounts of the HMG-CoA reductase inhibitor, the pharmaceutical formulation can be obtained as described in the earlier PCT application No. PCT/IB99/01749.

## Claims

1. Use of a substance capable of binding and/or neutralizing carbon dioxide in a pharmaceutical packaging which contains one or more formulations of a HMG-CoA reductase inhibitor selected from the group consisting of pravastatin, atorvastatin, fluvastatin, cerivastatin and pharmaceutically acceptable salts thereof, wherein said substance is contained in an element of a package separate from the HMG-CoA reductase inhibitor and said substance is selected from the group consisting of alkali metal hydroxides, alkali metal carbonates, alkali metal hydrogen carbonates and alkali metal superoxides, activated carbon, zeolites, activated aluminium oxide and Fuller's earth for stabilizing the HMG-CoA reductase inhibitor.

2. The use according to claim 1, wherein said substance is potassium superoxide.

3. The use according to claim 1, wherein said substance is KOH, NaOH or LiOH.

4. The use according to claim 1, wherein said substance is a zeolite which binds carbon dioxide.

## Patentansprüche

1. Verwendung einer Substanz, die Kohlendioxid binden und/oder neutralisieren kann, in einer pharmazeutischen Verpackung, welche eine oder mehrere Formulierungen eines HMG-CoA-Reduktaseinhibitors ausgewählt aus der Gruppe bestehend aus Pravastatin, Atorvastatin, Fluvastatin, Cerivastatin und pharmazeutisch akzeptablen Salzen davon enthält, wobei die Substanz in einem Element einer Verpackung getrennt von dem HMG-CoA-Reduktaseinhibitor enthalten ist, und die Substanz ausgewählt ist aus der Gruppe bestehend aus Alkalimetallhydroxiden, Alkalimetallcarbonaten, Alkalimetallhydrogencarbonaten und Alkalimetallhyperoxiden, Aktivkohle, Zeolithen, aktiviertem Aluminiumoxid und Fuller-Erde für die Stabilisierung des HMG-CoA-Reduktaseinhibitors.

2. Verwendung nach Anspruch 1, wobei die Substanz Kaliumhyperoxid ist.

3. Verwendung nach Anspruch 1, wobei die Substanz KOH, NaOH oder LiOH ist.

4. Verwendung nach Anspruch 1, wobei die Substanz ein Zeolith ist, welcher Kohlendioxid bindet.

## Revendications

1. Utilisation d'une substance capable de se lier avec le dioxyde de carbone et/ou de neutraliser celui-ci au sein d'un conditionnement pharmaceutique qui contient une ou plusieurs formulations d'un inhibiteur de la HMG-CoA réductase choisi dans le groupe constitué par la pravastatine, l'atorvastatine, la fluvastatine, la cérivastatine et les sels pharmaceutiquement acceptables de celles-ci, dans laquelle ladite substance est contenue dans un élément d'un conditionnement séparé de l'inhibiteur de la HMG-CoA réductase et ladite substance est choisie dans le groupe constitué par des hydroxydes de métaux alcalins, des carbonates de métaux alcalins, des hydrogénocarbonates de métaux alcalins et des superoxydes de métaux alcalins, un charbon actif, des zéolithes, un oxyde d'aluminium activé et une terre à foulon pour stabiliser l'inhibiteur de la HMG-CoA réductase.

2. Utilisation selon la revendication 1, dans laquelle ladite substance est le superoxyde de potassium.

3. Utilisation selon la revendication 1, dans laquelle ladite substance est le KOH, le NaOH ou le LiOH.

4. Utilisation selon la revendication 1, dans laquelle ladite substance est une zéolite qui se lie au dioxyde de carbone.
